# EUROPEAN PATENT APPLICATION

(11) **EP 4 368 223 A1**
(43) Date of publication of application: **15.05.2024**
(21) Application number: 22876606.9
(22) Date of filing: 10.05.2022
(51) Int. Cl.: A61M 5/30, A61M 5/315, A61M 5/31

(54) **NEEDLE-FREE INJECTION SYSTEM HAVING ADJUSTABLE DRUG INJECTION ATTRIBUTES**

(30) Priority: 30.09.2021 KR 20210129752
(71) Applicant: Baz Biomedic Co., Ltd., Seoul 08826 (KR)
(72) Inventor: KIM, Jung Kook, Daejeon 34817 (KR); HAM, Hwi Chan, Seoul 08732 (KR); LEE, Sung Hun, Gapyeong-gun, Gyeonggi-do 12428 (KR)
(74) Representative: Vossius & Partner Patentanwälte Rechtsanwälte mbB
(86) International application number: PCT/KR2022/006671
(87) International publication number: WO 2023/054831

(57) **Abstract**

The present invention is configured so that a user can adjust injection attributes including drug injection mode, injection amount, injection depth, and injection speed, and thus has the advantage that convenience of use can be further improved. In addition, the user can set desired injection attributes through a user interface, and thus convenience can be improved. Moreover, a number of outputs, a period, an amplitude, an output time, and an output off time of a pulse applied to a solenoid coil can be adjusted to adjust the injection attributes of a drug to those desired by the user.

## Description

### Technical field

The present invention relates to a needleless syringe system, and more particularly, to a needleless syringe system having adjustable drug injection attributes including an injection mode, an injection amount, an injection depth, and a number of injections of a drug.

### Background art

In general, a syringe is a device for injecting a medicinal solution into the tissue of an organism. The syringe includes a needle inserted into the body, a syringe cylinder in which the medicinal solution is accommodated, and a piston that reciprocates inside the syringe cylinder and pushes the medicinal solution with the needle. The needle is punctured to allow a drug to be injected when injected.

Recently, in order to relieve the fear of the needle of the syringe and to prevent infection due to the needle, research and development on the syringe without the needle has been actively carried out.

However, because the existing needleless syringe is configured to inject a predetermined amount of a drug into only one part of the skin at a time, damage to the skin tissue may occur.

In addition, because discomfort such as reloading after one injection follows, there is a limitation in that the needleless syringe cannot be used to evenly inject the drug multiple times into a large area of the skin in the field of skin care and the like.

### Detailed description of the invention

### Technical problem

The objective of the present invention is to provide a needleless syringe system in which a user can inject a drug while adjusting injection attributes of the drug so that convenience of use can be improved.

### Technical solution

According to an aspect of the present invention, there is provided a needleless syringe system having adjustable drug injection attributes, the needleless syringe system including: a solenoid coil wound on an outer circumferential surface of the body; a cylinder coupled to the body to be in communication with an open front surface of the body; a drug accommodating portion, which is formed on a front inner side of the cylinder and in which a drug injected from an outside is accommodated; a nozzle portion, which is provided in front of the cylinder and through which the drug accommodated in the drug accommodating portion is discharged forward; a moving magnetic body, which is provided inside the body and moves forward by a magnetic force generated when power is supplied to the solenoid coil; a piston, which is provided inside the cylinder and moves forward by an impulse applied by the moving magnetic body when the moving magnetic body moves forward, to pressurize the drug in the drug accommodating portion; a pulse generator applying a pulse to the solenoid coil; a user interface that allows a user to set injection attributes including at least one of an injection mode, an injection amount, an injection depth, and an injection speed of the drug discharged through the nozzle portion and injected into the skin; and a control unit adjusting at least one of a number of outputs N, a period T, an amplitude, an output time tₒₙ, and an output off time t_{off} at which no pulse is output, of a pulse applied to the solenoid coil from the pulse generator according to the injection attributes set through the user interface to control the injection attributes.

The user interface in which an injection mode of the drug is divided into a single shot mode in which the drug is injected the drug once, a burst mode in which the drug is injected at a set number of times and a continuous shot mode in which injection and blocking of the drug is continuously repeated and is displayed, may allow the user to select and set the injection mode of the drug.

When the single shot mode is set through the user interface, the control unit may set a number of outputs of the pulse once, may adjust an amplitude of the pulse according to an injection depth of the drug set through the user interface and may adjust an output time tₒₙ of the pulse according to an injection amount of the drug.

When the burst mode is set through the user interface, the control unit may set a number of outputs of the pulse to the set number of times, may adjust an amplitude of the pulse according to an injection depth of the drug set through the user interface, may adjust an output time tₒₙ of the pulse according to an injection amount of the drug, adjusts a period of the pulse according to an injection speed of the drug, and may set a time obtained by subtracting the output time tₒₙ of the pulse from the period of the pulse, to the output off time t_{off}.

When the continuous shot mode is set through the user interface, the control unit may adjust an amplitude of the pulse according to an injection depth of the drug set through the user interface, may adjust an output time tₒₙ of the pulse according to an injection amount of the drug, may adjust a period of the pulse according to an injection speed of the drug, and may set a time obtained by subtracting the output time tₒₙ of the pulse from the period of the pulse, to the output off time t_{off}.

The user interface may allow the user to increase/decrease and to set the injection amount of the drug within a set range, and the control unit may increase the output tine tₒₙ of the pulse as the injection amount of the drug set through the user interface is increased.

The user interface in which the injection depth of the drug is divided into a plurality of levels at a set depth interval, may allow the user to select one of the plurality of levels and set the selected level, and the control unit may increase the amplitude of the pulse as the injection depth of the drug set through the user interface is increased.

The user interface in which the injection speed of the drug is displayed in a number of injections per second of the drug, may allow the user to set the injection speed by increasing/decreasing the number of injections per second of the drug, and as the injection speed of the drug set through the user interface is increased, the control unit may decrease the period of the pulse and may decrease the output off time t_{off}.

The user interface may include a selection unit through which the user selects each of the injection mode, the injection amount, the injection depth and the injection speed, and a display unit on which each of the injection mode, the injection amount, the injection depth and the injection speed selected by the user is displayed.

The user interface may include a terminal capable of wired or wireless communication with the control unit.

The needleless syringe system may further include an elastic member for a piston, which is provided inside of at least one of the body and the cylinder and applies an elastic force to the piston in a direction in which the piston moves backward, when the supply of current to the solenoid coil is cut off.

The needleless syringe system may further include a nozzle portion opening/closing valve that is provided to open/close a passage hole between the nozzle portion and the drug accommodating portion, is pushed by a fluidic pressure applied by the drug from the drug accommodating portion during forward movement of the piston to open the passage hole, and is elastically restored when the fluidic pressure is released to close the passage hole.

The needleless syringe system may further include a cooling chamber that is provided to surround an outside of the solenoid coil on an outside of the body and absorbs and cools heat generated in the solenoid coil through a cooling fluid.

According to another aspect of the present invention, there is provided a needleless syringe system having adjustable drug injection attributes, the needleless syringe system including: a solenoid coil wound on an outer circumferential surface of the body; a cylinder coupled to the body to be in communication with an open front surface of the body; a drug accommodating portion, which is formed on a front inner side of the cylinder and in which a drug injected from an outside is accommodated; a nozzle portion, which is provided in front of the cylinder and through which the drug accommodated in the drug accommodating portion is discharged forward; a moving magnetic body, which is provided inside the body and moves forward by a magnetic force generated when power is supplied to the solenoid coil; a piston, which is provided inside the cylinder and moves forward by an impulse applied by the moving magnetic body when the moving magnetic body moves forward, to pressurize the drug in the drug accommodating portion; a nozzle portion opening/closing valve that is provided to open/close a passage hole between the nozzle portion and the drug accommodating portion, is pushed by a fluidic pressure applied by the drug from the drug accommodating portion during forward movement of the piston to open the passage hole and is elastically restored when the fluidic pressure is released to close the passage hole; a cooling chamber that is provided to surround an outside of the solenoid coil on an outside of the body and absorbs and cools heat generated in the solenoid coil through a cooling fluid; a pulse generator applying a pulse to the solenoid coil; a user interface that allows a user to set injection attributes including at least one of an injection mode, an injection amount, an injection depth, and an injection speed of the drug discharged through the nozzle portion and injected into the skin; and a control unit adjusting at least one of a number of outputs N, a period T, an amplitude, an output time tₒₙ, and an output off time t_{off} at which no pulse is output, of a pulse applied to the solenoid coil from the pulse generator according to the injection attributes set through the user interface to control the injection attributes, wherein the user interface in which an injection mode of the drug is divided into a single shot mode in which the drug is injected the drug once, a burst mode in which the drug is injected at a set number of times and a continuous shot mode in which injection and blocking of the drug is continuously repeated and is displayed, allows the user to select and set the injection mode of the drug, and when the single shot mode is set through the user interface, the control unit sets a number of outputs of the pulse once, adjusts an amplitude of the pulse according to an injection depth of the drug set through the user interface and adjusts an output time tₒₙ of the pulse according to an injection amount of the drug, and when the burst mode is set through the user interface, the control unit sets a number of outputs of the pulse to the set number of times, adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}, and when the continuous shot mode is set through the user interface, the control unit adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}.

According to another aspect of the present invention, there is provided a needleless syringe system having adjustable drug injection attributes, the needleless syringe system including: a solenoid coil wound on an outer circumferential surface of the body; a cylinder coupled to the body to be in communication with an open front surface of the body; a drug accommodating portion, which is formed on a front inner side of the cylinder and in which a drug injected from an outside is accommodated; a nozzle portion, which is provided in front of the cylinder and through which the drug accommodated in the drug accommodating portion is discharged forward; a piston, which is provided inside the body and moves forward by a magnetic force generated when power is supplied to the solenoid coil, to pressurize the drug in the drug accommodating portion; a pulse generator applying a pulse to the solenoid coil; a user interface that allows a user to set injection attributes including at least one of an injection mode, an injection amount, an injection depth, and an injection speed of the drug discharged through the nozzle portion and injected into the skin; and a control unit adjusting at least one of a number of outputs N, a period T, an amplitude, an output time tₒₙ, and an output off time t_{off} at which no pulse is output, of a pulse applied to the solenoid coil from the pulse generator according to the injection attributes set through the user interface to control the injection attributes.

According to another aspect of the present invention, there is provided a needleless syringe system having adjustable drug injection attributes, the needleless syringe system including: a drug accommodating portion in which a drug injected from an outside is accommodated; a nozzle portion through which the drug accommodated in the drug accommodating portion is discharged forward; a solenoid mechanism including a piston that pressurizes the drug in the drug accommodating portion repeatedly while repeating forward movement and backward movement so that the drug accommodated in the drug accommodating portion is discharged through the nozzle portion, a solenoid coil that forms an electromagnetic force to move the piston forward, and a pulse generator that applies a pulse to the solenoid coil; and a control unit controlling the pulse generator to adjust an injection amount and an injection depth of the drug discharged through the nozzle portion and injected into the skin.

According to another aspect of the present invention, there is provided a needleless syringe system having adjustable drug injection attributes, the needleless syringe system including: a drug accommodating portion in which a drug injected from an outside is accommodated; a nozzle portion through which the drug accommodated in the drug accommodating portion is discharged forward; a solenoid mechanism including a piston that pressurizes the drug in the drug accommodating portion repeatedly while repeating forward movement and backward movement so that the drug accommodated in the drug accommodating portion is discharged through the nozzle portion, a solenoid coil that forms an electromagnetic force to move the piston forward, and a pulse generator that applies a pulse to the solenoid coil; a user interface that allows a user to set an injection amount and an injection depth of the drug discharged through the nozzle portion and injected into the skin; and a control unit controlling the pulse generator based on setting information input to the user interface.

According to another aspect of the present invention, there is provided a needleless syringe system having adjustable drug injection attributes, the needleless syringe system including: a cylinder having a drug accommodating portion in which a drug injected from an outside is accommodated, formed therein; a nozzle portion, which communicates with the drug accommodating portion of the cylinder and through which the drug accommodated in the drug accommodating portion is discharged forward; a drug pressurizing portion pressurizing the drug in the drug accommodating portion to flow the drug toward the nozzle portion; a driving unit driving the drug accommodating portion; a pulse generator that applies a pulse to the driving unit; a user interface that allows a user to set injection attributes including at least one of an injection mode, an injection amount, an injection depth, and an injection speed of the drug discharged through the nozzle portion and injected into the skin; and a control unit adjusting at least one of a number of outputs N, a period T, an amplitude, an output time tₒₙ, and an output off time t_{off} at which no pulse is output, of a pulse applied to the driving unit from the pulse generator according to the injection attributes set through the user interface, to control the injection attributes.

### Effects of the invention

The present invention is configured so that a user can adjust injection attributes including an injection mode, an injection amount, an injection depth, and an injection speed of a drug and thus has the advantage that convenience of use can be further improved.

In addition, the user can set desired injection attributes through a user interface, and thus convenience can be improved.

Moreover, a number of outputs, a period, an amplitude, an output time, and an output off time of a pulse applied to a solenoid coil can be adjusted so that the user can adjust desired drug injection attributes.

### Description of the drawings

FIG. 1 is a view showing a forward movement state of a piston of a needleless syringe according to an embodiment of the present invention.
FIG. 2 is a view showing a backward movement state of the piston of the needleless syringe according to an embodiment of the present invention.
FIG. 3 is a block diagram schematically showing the configuration of a needleless syringe system having adjustable drug injection attributes according to an embodiment of the present invention.
FIG. 4 shows an example of a user interface in a needleless syringe system according to an embodiment of the present invention.
FIG. 5 is a view showing an example of a waveform of a pulse when an injection mode of the needless syringe system according to an embodiment of the present invention is a single shot mode.
FIG. 6 is a view showing a first example of a waveform of a pulse when the injection mode of the needless syringe system according to an embodiment of the present invention is a 4 times burst mode and a first injection amount and a first injection speed are set.
FIG. 7 is a view showing a second example of a waveform of a pulse when the injection mode of the needless syringe system according to an embodiment of the present invention is a 4 times burst mode and a second injection amount and a first injection speed are set.
FIG. 8 is a view showing a third example of a waveform of a pulse when the injection mode of the needless syringe system according to an embodiment of the present invention is a 4 times burst mode and a first injection amount and a second injection speed are set.

### Mode of the invention

Hereinafter, an embodiment of the present invention will be described with reference to the accompanying drawings.

A needless syringe system according to an embodiment of the present invention is a system capable of adjusting injection attributes of a needless syringe that injects a drug into the skin by pressurizing the drug without using a syringe needle.

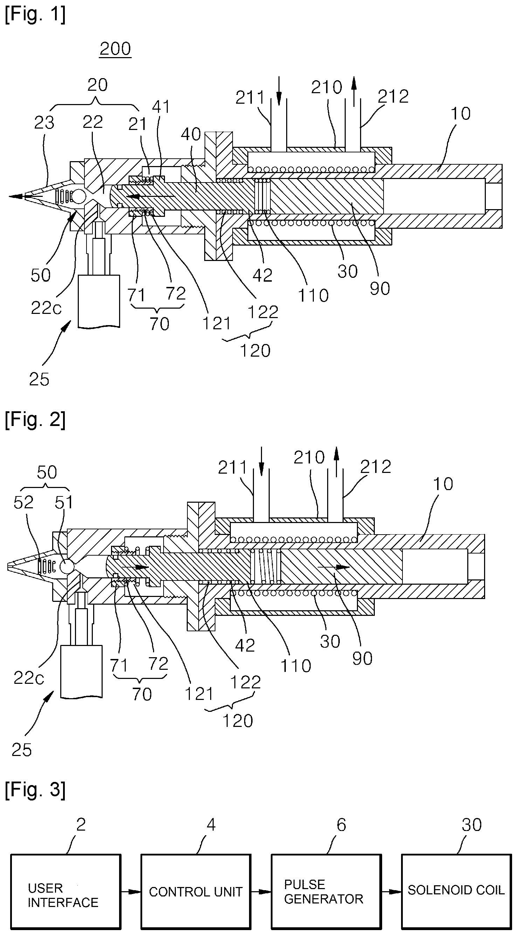

FIG. 1 is a view showing a forward movement state of a piston of a needleless syringe according to an embodiment of the present invention. FIG. 2 is a view showing a backward movement state of the piston of the needleless syringe according to an embodiment of the present invention.

Referring to FIGS. 1 and 2, a needleless syringe according to the embodiment of the present invention includes a body 10, a cylinder 20, a solenoid coil 30, a moving magnetic body 90, a piston 40, a nozzle portion opening/closing valve 50, an elastic member 110 for a moving magnetic body, an elastic member 120 for a piston, a blocker 70, and a cooling chamber 210.

The needleless syringe is an impact type syringe in which, when the moving magnetic body 90 moves forward by a magnetic force generated by the solenoid coil 30, the moving magnetic body 90 collides with the piston 40 to move the piston 40 forward.

The body 10 is formed in a hollow shape and is long formed in a longitudinal direction. The front surface of the body 10 is formed to be open.

The cylinder 20 is formed in a hollow shape and is coupled to the body 10 to be in communication with the open front surface of the body 10. A cylinder main hole 21 and a drug accommodating portion 22 are formed inside the cylinder 20, and a nozzle portion 31 is provided in front of the cylinder 20.

The cylinder main hole 21 is formed in the rear of an inner side of the cylinder 20, and a thread is formed in such a way that the front end of the body 10 is inserted into at least a part of the cylinder 20 to be screw-coupled to each other.

The drug accommodating portion 22 is a hole which is formed in the front inner side of the cylinder 20 and in which the drug injected from the outside is accommodated and the drug pressurized by the piston 40 passes. The drug accommodating portion 22 is formed to have a reduced cross-sectional area compared to the cylinder main hole 21. The cross-sectional area of the drug accommodating portion 22 may be set according to a drug injection amount. The drug accommodating portion 22 is formed in a shape of a diverging nozzle including a reduced portion whose cross-sectional area is gradually reduced toward the front, and an enlarged portion that extends from the reduced portion and increases in cross-sectional area again. A drug supply hole 22c for supplying the drug from the outside by a pressure difference generated when the piston 40 moves backward is formed in the reduced portion. A drug filling device 25 is coupled to the drug supply hole 22c.

The nozzle portion 23 is provided in front of the cylinder 20 and has a hole through which the drug accommodated in the drug accommodating portion 22 is discharged forward. The nozzle portion 23 is formed to be in communication with the drug accommodating portion 22 and to have a gradually decreasing cross-sectional area, and injects the drug accommodated in the drug accommodating portion 22. The nozzle portion 23 may also be integrally formed on an end portion of the cylinder 20 and may also be coupled to the end portion of the cylinder 20 to be replaceable. In the present embodiment, a case where the cylinder 20 is formed by combining a first block in which the cylinder main hole 21 and the drug accommodating portion 22 are formed, with a second block in which the nozzle portion 23 is formed, will be described. However, the present invention is not limited thereto, and the first block and the second block may also be integrally formed.

The solenoid coil 30 is a coil that is wound on the front of an outer circumferential surface of the body 10 and to which a current is applied when the piston 40 moves forward. The solenoid coil 30 generates a magnetic force in a direction in which the moving magnetic body 90 moves forward, when a current is applied to the solenoid coil 30, so as to move the moving magnetic body 90 forward.

The piston 40 is long inserted into the body 10 and the cylinder 20 in the longitudinal direction and pushes the drug accommodated in the drug accommodating portion 22. The piston 40 is provided separately from the moving magnetic body 90 inside the body 10 and is inserted in front of the moving magnetic body 90. The piston 40 moves forward by an impulse applied by the moving magnetic body 90 when the moving magnetic body 90 moves forward, to pressurize the drug in the drug accommodating portion 22 toward the nozzle portion 23. A first flange portion 41 protruding in a radial direction is formed on the outer circumferential surface of the front of the piston 40 located inside the cylinder 20. The first flange portion 41 is blocked by a length adjustment blocker 72 to be described later during the forward movement of the piston 40 so that a forward movement distance of the piston 40 is limited. A second flange portion 42 protruding in a radial direction is formed on the outer circumferential surface of the rear of the piston 40 located inside the body 10.

The moving magnetic body 90 is not a permanent magnet, but is formed of a material that temporarily has magnetism by a magnetic field generated when a current is applied to the solenoid coil 30 and disappears when the external magnetic field disappears. A case where the moving magnetic body 90 is an iron core, will be described.

The elastic member 110 for the moving magnetic body is long provided between the moving magnetic body 90 and the piston 40 inside the body 10 in a longitudinal direction of the body 10. The elastic member 110 for the moving magnetic body applies an elastic force to the moving magnetic body 110 in a direction in which the moving magnetic body 90 moves backward. The elastic member 110 for the moving magnetic body is a first coil spring that is compressed when the moving magnetic body 90 moves forward and applies an elastic force to the moving magnetic body 90 in a backward movement direction of the moving magnetic body 90. One end of the elastic member 110 for the moving magnetic body may be coupled to the rear end of the piston 40, and the other end of the elastic member 110 for the moving magnetic body may be coupled to the front end of the moving magnetic body 90.

The nozzle portion opening/closing valve 50 is provided to open and close a passage hole between the nozzle portion 23 and the drug accommodating portion 22. The nozzle portion opening/closing valve 50 is pushed by a fluidic pressure applied by the drug accommodated in the drug accommodating portion 22 during the forward movement of the piston 40 to open the passage hole, and is elastically restored when the fluidic pressure is released to close the passage hole.

The nozzle portion opening/closing valve 50 includes a ball 51 installed in the passage hole, and an elastic member 52 that is installed in the nozzle portion 23 to provide an elastic force in a direction of the ball 51 toward the drug accommodating portion 22. The ball 51 is formed to fit into the enlarged portion. In the present embodiment, the nozzle portion opening/closing valve 50 has been described as an example of a ball valve. However, the present invention is not limited thereto, and various valves such as a duckbill valve, a plate check valve, an electric control valve, and the like may be used as the nozzle portion opening/closing valve 50.

The elastic member 120 for the piston is an elastic member that is provided inside of at least one of the body 10 and the cylinder 20 and applies an elastic force to the piston 40 in a backward movement direction of the piston 40 when the supply of current to the solenoid coil 30 is cut off. A case where the elastic member 120 for the piston includes a second coil spring 121 and a third coil spring 122 coupled to the outer circumferential surface of the piston 40, will be described.

The second coil spring 121 is extrapolated to the piston 40 and has both ends provided between the cylinder 20 and the first flange portion 41. The second coil spring 121 is compressed by the first flange portion 41 when the piston 40 moves forward, and applies an elastic force to the first flange portion 41 in a backward movement direction of the piston 40 when the piston 40 moves backward.

The third coil spring 122 is extrapolated to the piston 40 and has both ends provided between the body 10 and the second flange portion 42. The second coil spring 122 is compressed by the second flange portion 42 when the piston 40 moves forward, and applies an elastic force to the second flange portion 42 in a backward movement direction of the piston 40 when the piston 40 moves backward.

The blocker 70 is detachably coupled between the cylinder 20 and the piston 40. The blocker 70 includes a fixed blocker 71 coupled to and fixed to the cylinder main hole 21, and a length adjustment blocker 72 that is screwed to an inner circumferential surface of the fixed blocker 71 and adjusts a length at which the length adjustment blocker 72 is coupled to the fixed blocker 71.

The fixed blocker 71 has a female thread formed on an inner circumferential surface of the fixed blocker 71 and formed in a ring shape.

The length adjustment blocker 72 has a male thread on an outer circumferential surface of the length adjustment blocker 72 and formed in a ring shape. A predetermined interval is formed between the length adjustment blocker 72 and the piston 40, and the piston 40 may pass through the inside of the length adjustment blocker 72 to move forward and backward.

The cooling chamber 210 is a cooling unit that is detachably coupled to the outside of the body 10, is provided to surround the solenoid coil 30 and cools heat generated in the solenoid coil 30 through a cooling fluid. A cooling fluid supply pipe 211 and a cooling fluid discharge pipe 212 are coupled to the cooling chamber 210.

The cooling fluid supply pipe 211 is a flow path for supplying the cooling fluid from the outside to the cooling chamber 210. The cooling fluid discharge pipe 212 is a flow path for discharging the cooling fluid of the first cooling chamber 211 to the outside. An opening/closing valve (not shown) may be provided in the cooling fluid supply pipe 211 and the cooling fluid discharge pipe 212, respectively.

The cooling chamber 210 is provided so that heat of the solenoid coil 30 is absorbed to maintain a constant temperature and thus, the magnetic force can be prevented from being weakened by the heat generated by the solenoid coil 30.

In the present embodiment, a cooling fluid is used to cool the solenoid coil 30, and water or air is used as the cooling fluid. However, the present invention is not limited, and a conduction cooling method or the like may be used.

A piston cover (not shown) may be provided between the cylinder 20 and the piston 40. The piston cover (not shown) is fixedly installed at the cylinder 20. The piston cover 810 is provided inside the cylinder 20 and is disposed to cover the end portion of the piston 40. The piston cover (not shown) may be formed of an elastic material to be elongated forward by the piston 40 when the piston 40 moves forward and to be restored when the piston 40 moves backward.

FIG. 3 is a block diagram schematically showing the configuration of a needleless syringe system having adjustable drug injection attributes according to an embodiment of the present invention.

Referring to FIG. 3, the needleless syringe system further includes a user interface 2, a control unit 4, and a pulse generator 6.

In the present invention, a case where the user interface 2, the control unit 4 and the pulse generator 6 are provided separately from the needleless syringe, will be described, but the present invention is not limited thereto, and the user interface 2, the control unit 4, and the pulse generator 6 may also be provided integrally with the needleless syringe.

The user interface 2 allows the user to set injection attributes of the drug of the needleless syringe. The user interface 2 includes a terminal capable of wired or wireless communication with the control unit 4. The terminal may include a computer, a smartphone, a tablet personal computer (PC), or the like.

The injection attributes of the drug include at least one of an injection mode of the drug, an injection amount of the drug, an injection depth of the drug, and an injection speed of the drug.

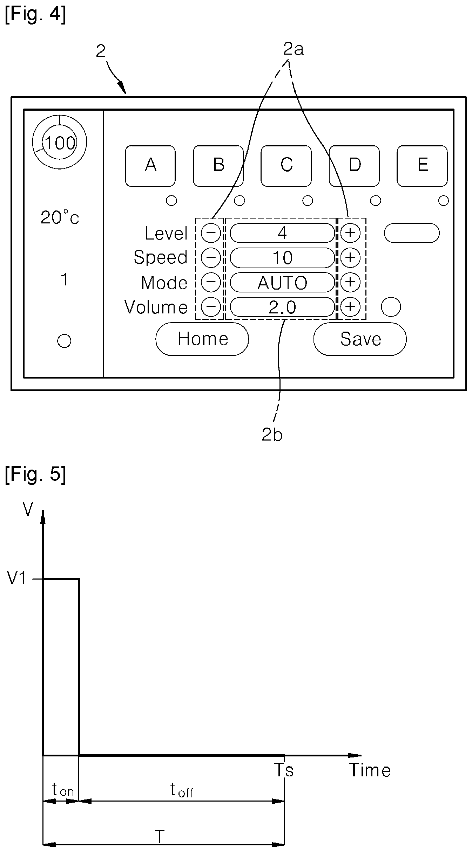

Referring to FIG. 4, the user interface 2 includes a selection unit 2a through which the user selects each of the injection mode (Mode), the injection amount (Volume), the injection depth (Level) and an injection speed (Speed), and a display unit 2b on which each of the injection mode, the injection amount, the injection depth and the injection speed is displayed. That is, the selection unit 2a includes an injection mode selection unit, an injection amount selection unit, an injection depth selection unit, and an injection speed selection unit. In addition, the user interface 2 further includes a storage button through which the injection mode, the injection amount, the injection depth and the injection speed input from the user may be stored.

The user interface 2 will be described as an example in which the injection mode of the drug is divided into three modes and displayed. That is, the injection mode includes a single shot mode in which the drug is injected only once when a switch for operating the needleless syringe is turned on, a burst mode in which the drug is injected a set number of times when the switch is turned on, and a continuous shot mode in which injection and blocking of the drug are continuously repeated. The user may select and set one of the single shot mode, the burst mode, and the continuous shot mode through the injection mode selection unit.

In addition, the user interface 2 allows the user to increase/decrease and set the injection amount of the drug in a set range. In the present embodiment, a case where the injection amount is adjustable in the range of about 0.1 *µ*ℓ to 2.0 *µ*ℓ*.* The user may select and set the injection amount through the injection amount selection unit.

In addition, the user interface 2 in which the injection depth of the drug is divided into a plurality of levels at a set depth interval and displayed, allows the user to select one of the plurality of levels and set the selected level. In the present embodiment, a case where the injection depth is adjustable in the range of about 0.2 mm to 5.0 mm, will be described. The user may select and set the injection depth through the injection depth selection unit.

In addition, the user interface 2 allows the user to increase/decrease and set the injection speed of the drug by displaying the injection speed of the drug in a number of injections per second of the drug. That is, a number of injections per second of the drug is a frequency of a pulse to be described later. In the present embodiment, a case where the injection speed is about 1 to 30 Hz, will be described.

The pulse generator 6 applies a pulse signal to the solenoid coil 30. The pulse generator 6 is included in a power supply unit for supplying power to the solenoid coil 30 from an external power supply source (not shown).

The control unit 4 controls the injection attributes by adjusting the waveform of the pulse applied to the solenoid coil according to injection attributes set through the user interface 2. That is, the control unit 4 adjusts a number of outputs N of the pulse, a period of the pulse, an amplitude of the pulse, an output time tₒₙ of the pulse, and an output off time t_{off} at which no pulse is output, of the pulse to transmit them to the pulse generator 6.

The operation of the needleless syringe system according to the embodiment of the present invention having the above configuration will be described as follows.

The user who uses the needleless syringe may set desired injection mode (Mode), injection amount (Volume), injection depth (Level), and injection speed (Speed) of the drug through the user interface 2. When the user sets each of the injection attributes through the user interface 2, an input signal for the injection attributes is transmitted to the control unit 4.

FIG. 5 is a view showing an example of a waveform of a pulse when an injection mode of the needleless syringe system according to an embodiment of the present invention is a single shot mode.

Referring to FIG. 5, a case where the user sets the injection mode to the single shot mode through the user interface 2, the injection amount of the drug is set to a first injection amount and the injection depth is set to a first level, will be described.

When the single shot mode is set, the control unit 4 sets a number of outputs N of the pulse output when a switch of the needleless syringe is turned on, to once. That is, a number of outputs N of the pulse is set according to the injection mode.

In addition, when the injection amount is set to the first injection amount through the user interface 2, the control unit 4 sets the output time tₒₙ of the pulse to a first output time tₒₙ1 according to the first injection amount. That is, the control unit 4 in which data about the relationship between the injection amount and the output time tₒₙ of the pulse are previously stored, may derive the output time tₒₙ suitable for a set injection amount when the injection amount is set. The output time of the pulse is time at which the pulse is output once with an amplitude to be described later. As the injection amount is set larger, the output time tₒₙ of the pulse is set longer, and as the injection amount is decreased, the output time tₒₙ of the pulse is set shorter.

In addition, when the injection depth is set to the first injection depth through the user interface 2, the control unit 4 sets the amplitude V of the pulse to a first amplitude V1 according to the first injection depth. That is, the control unit 4 in which data about the relationship between the injection depth and the amplitude V of the pulse are previously stored, may derive an amplitude V suitable for a set injection depth when the injection depth is set. Here, the amplitude of the pulse corresponds to the magnitude of a voltage. As the injection depth is set larger, the amplitude of the pulse is set larger, and as the injection depth is decreased, the amplitude of the pulse is set smaller.

Since the drug is injected only once in the single shot mode, the injection speed of the drug is not adjusted. That is, the user interface 2 may deactivate the selection unit for setting the injection speed when the single shot mode is set.

As described above, when the user sets the single shot mode and sets the injection depth of the drug and the injection amount of the drug, the control unit 4 sets the amplitude V of the pulse and the output time tₒₙ of the pulse, respectively, according to the injection depth and the injection amount.

The pulse generator 6 generates the pulse with a first amplitude V and a first output time tₒₙ1 set by the control unit 4, and the pulse is applied to the solenoid coil 30.

Referring to FIG. 5, it can be known that, in the single shot mode, the pulse is output once and is output during the first output time tₒₙ1 and is generated with a waveform having the first amplitude V1.

When the pulse is applied to the solenoid coil 30, time at which the solenoid coil 30 generates a magnetic force according to the output time tₒₙ of the pulse, is adjusted, and a collision time at which the moving magnetic body 90 collides with the piston 40, is adjusted.

As the output time tₒₙ of the pulse is increased, the collision time of the moving magnetic body 90 and the piston 40 is increased, and time at which the piston 40 moves forward, is increased and thus, an ejection time of the drug is increased, and the injection amount of the drug is increased. Thus, as the output time of the pulse is increased, the injection amount of the drug is increased, and as the output time of the pulse is decreased, the injection amount of the drug is decreased. The output time of the pulse is increased/decreased so that the user can adjust a desired drug injection amount.

In addition, the size of the magnetic force generated in the solenoid coil 30 is adjusted according to the amplitude V of the pulse, and the forward movement speed of the moving magnetic body 90 is adjusted according to the size of the magnetic force. As the amplitude of the pulse is increased, the forward movement speed of the moving magnetic body 90 is increased, and the injection depth of the drug is increased. Thus, as the amplitude of the pulse is increased, the injection depth of the drug is increased, and as the amplitude of the pulse is decreased, the injection depth of the drug is decreased. That is, the amplitude of the pulse is increased/decreased so that the user can adjust a desired drug injection depth.

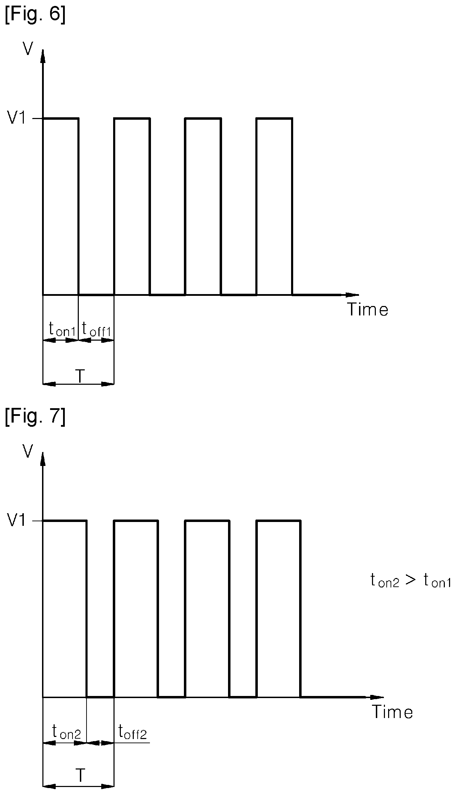

Meanwhile, FIG. 6 is a view showing a first example of a waveform of a pulse when the injection mode of the needless syringe system according to an embodiment of the present invention is a 4 times burst mode and a first injection amount and a first injection speed are set.

Referring to FIG. 6, a case where the user sets the injection mode to the burst mode, the injection amount of the drug to a first injection amount, the injection depth to a first level and the injection speed to a first injection speed through the user interface 2, will be described.

When the burst mode is set, the user sets a number of injections of the drug together. For example, when the burst mode is set, one of a 2 times burst mode, a 3 times burst mode, and a 4 times burst mode is selected and set. Hereinafter, a case where the 4 times burst mode is set, will be described.

When the 4 times burst mode is set, the control unit 4 sets a number of outputs N of the pulse output when the switch of the needleless syringe is turned on, to 4 times. That is, when the switch is turned on once, the pulse is output 4 times so that the drug can be injected 4 times with one switching operation.

In addition, when the injection amount is set to the first injection amount through the user interface 2, the control unit 4 sets the output time tₒₙ of the pulse to the first output time tₒₙ1 according to the first injection amount. That is, the control unit 4 in which data about the relationship between the injection amount and the output time tₒₙ of the pulse are previously stored, may derive the output time tₒₙ suitable for the set injection amount when the injection amount is set. The output time tₒₙ of the pulse is time at which the pulse is output once with an amplitude to be described later. As the injection amount is set larger, the output time tₒₙ of the pulse is set longer.

In addition, when the injection depth is set to the first injection depth through the user interface 2, the control unit 4 sets an amplitude V of the pulse to a first amplitude V1 according to the first injection depth. That is, the control unit 4 in which data about the relationship between the injection depth and the amplitude V of the pulse are previously stored, may derive the amplitude V suitable for the set injection depth when the injection depth is set. Here, the amplitude of the pulse correspond to the magnitude of a voltage. As the injection depth is set larger, the amplitude of the pulse is set larger, and as the injection depth is decreased, the amplitude of the pulse is also set smaller.

In addition, when the injection speed is set to the first injection speed through the user interface 2, the control unit 4 sets the period T of the pulse according to the first injection speed. Here, since the injection speed is a number of injections per second of the drug, the period T of the pulse is set to a reciprocal number of the injection speed. For example, when the injection speed is set to 10, the period T of the pulse may be set to 1/10.

The control unit 4 sets the period T of the pulse according to the injection speed and sets the remaining time obtained by subtracting the pulse output time tₒₙ from the period T of the pulse, to an output off time t_{off} at which the pulse is not output, of the pulse. Here, a case where the output off time t_{off} of the pulse is set to a first output off time t_{off}1, will be described.

The control unit 4 may derive the period T of the pulse suitable for the set injection speed when the injection speed is set. Here, since the injection speed is a frequency, i.e., a number of injections per second of the drug, as the period of the pulse is decreased, a next pulse after the pulse is output, is output faster and thus, a number of injections per second of the drug is increased, and the injection speed is increased. Thus, as the injection speed is set faster, the period of the pulse is adjusted shorter, and as the injection speed is set slower, the period of the pulse is adjusted longer. In this case, when the period T of the pulse is adjusted according to the injection speed, the output time tₒₙ of the pulse changes according to the injection amount within a period of the pulse, the output off time t_{off} of the pulse that is the remaining time obtained by subtracting the pulse output time tₒₙ from the period T of the pulse, is adjusted.

As described above, when the user sets the burst mode and sets the injection depth of the drug, the injection amount of the drug and the injection speed of the drug, respectively, the control unit 4 sets the amplitude V of the pulse, the output time tₒₙ of the pulse, and the output off time t_{off} of the pulse, respectively, according to the injection depth, the injection amount, and the injection speed.

The pulse generator 6 generates the pulse according to a number of outputs of the pulse, a period of the pulse, the amplitude V of the pulse, the output time tₒₙ of the pulse, and the output off time t_{off} of the pulse, which are set by the control unit 4, and the pulse is applied to the solenoid coil 30.

Referring to FIG. 6, it can be known that, in the 4 times burst mode, the pulse is output 4 times and the pulse is output during the first output time tₒₙ1 and is not output during the first output off time t_{off}1 and is generated with a waveform of the first amplitude V1.

When the pulse is applied to the solenoid coil 30, a time at which the solenoid coil 30 generates a magnetic force according to the output time of the pulse, is adjusted, and a collision time at which the moving magnetic body 90 collides with the piston 40, is adjusted.

As the output time tₒₙ of the pulse is increased, the collision time of the moving magnetic body 90 and the piston 40 is increased and a forward movement time of the piston 40 is increased and thus the ejection time of the drug is increased, and the injection amount of the drug is increased. Thus, as the output time of the pulse is increased, the injection amount of the drug is increased, and as the output time of the pulse is decreased, the injection amount of the drug is decreased. The output time of the pulse is increased/decreased so that the user can adjust a desired drug injection amount.

In addition, the size of the magnetic force generated in the solenoid coil 30 is adjusted according to the amplitude V of the pulse, and the forward movement speed of the moving magnetic body 90 is adjusted according to the size of the magnetic force. As the amplitude of the pulse is increased, the forward movement speed of the moving magnetic body 90 is increased, and the injection depth of the drug is increased. Thus, as the amplitude of the pulse is increased, the injection depth of the drug is increased, and as the amplitude of the pulse is decreased, the injection depth of the drug is decreased. That is, the amplitude of the pulse is increased/decreased so that the user can adjust a desired drug injection depth.

Meanwhile, FIG. 7 is a view showing a second example of a waveform of a pulse when the injection mode of the needless syringe system according to an embodiment of the present invention is a 4 times burst mode and a second injection amount and a first injection speed are set.

Referring to FIG. 7, the user sets the injection mode to a 4 times burst mode through the user interface 2, wherein the injection amount of the drug is set to a second injection amount that is greater than the first injection amount. In addition, a case where the injection depth is set to a first level and the injection speed is set to a first injection speed, will be described.

When the 4 times burst mode is set, the control unit 4 sets a number of outputs of the pulse output when the switch of the needleless syringe is turned on, to 4 times. That is, when the switch is turned on once, the pulse is output 4 times and thus the drug can be injected 4 times with one switching operation.

In addition, when the injection amount is set to the second injection amount that is greater than the first injection amount through the user interface 2, the control unit 4 sets the output time tₒₙ of the pulse to a second output time tₒₙ2 according to the second injection amount. Since the second injection amount is greater than the first injection amount, the output time tₒₙ of the pulse is set to the second output time tₒₙ2 increased compared to the first output time tₒₙ1. That is, the control unit 4 in which data about the relationship between the injection amount and the output time tₒₙ of the pulse are previously stored, may derive the output time tₒₙ suitable for the set injection amount when the injection amount is set. As the injection amount is set larger, the output time of the pulse is set longer.

In addition, when the injection depth is set to the first injection depth through the user interface 2, the control unit 4 sets the amplitude V of the pulse to a first amplitude V1 according to the first injection depth. That is, the control unit 4 in which data about the relationship between the injection depth and the amplitude V of the pulse are previously stored, may derive the amplitude V suitable for the set injection depth when the injection depth is set.

In addition, when the injection speed is set to the first injection speed through the user interface 2, the control unit 4 sets the period T of the pulse according to the first injection speed.

The control unit 4 sets the period T of the pulse according to the injection speed and sets the remaining time obtained by subtracting the pulse output time tₒₙ from the period T of the pulse, to an output off time t_{off} at which the pulse is not output, of the pulse.

Thus, a case where the output off time t_{off} of the pulse is set to a second output off time t_{off}2 that is the remaining time obtained by subtracting the second output time tₒₙ2 of the pulse from the period T of the pulse, will be described. The second output off time t_{off}2 is a time that is shorter than the first output off time t_{off}2. When the injection amount is set to a second injection amount that is greater than the first injection amount, an output time tₒₙ of the pulse is set to the second output time tₒₙ2 that is longer than the first output time tₒₙ1, but the injection speed is the same and thus, the period T of the pulse is the same and thus, the second output off time t_{off}2 that is the remaining time obtained by subtracting the second output time tₒₙ2 from the period T of the pulse, is set to the output off time t_{off} of the pulse.

The control unit 4 may derive the period T of the pulse suitable for the set injection speed when the injection speed is set. Here, since the injection speed is a frequency, i.e., a number of injections per second of the drug, as the period of the pulse is decreased, a next pulse after the pulse is output, is output faster and thus, a number of injections per second of the drug is increased and the injection speed is increased. Thus, the injection speed is set in such a way that, as the injection speed is set faster, the period of the pulse is adjusted to be shorter and as the injection speed is set slower, the period of the pulse is adjusted to be longer. In this case, when the period T of the pulse is adjusted according to the injection speed, the output time tₒₙ of the pulse within the period of the pulse changes according to the injection amount and thus, the output off time t_{off} of the pulse that is the remaining time obtained by subtracting the pulse output time tₒₙ from the period T of the pulse, is adjusted.

As described above, when the user sets the 4 times burst mode, the injection depth of the drug, the injection amount of the drug and the injection speed of the drug, respectively, the control unit 4 sets each of the amplitude V of the pulse, the output time tₒₙ of the pulse, and the output off time t_{off} of the pulse according to the injection depth, the injection amount, and the injection speed.

The pulse generator 6 generates a pulse according to a number of outputs of the pulse, the amplitude V of the pulse, the output time tₒₙ of the pulse, and the output off time t_{off} of the pulse, which are set by the control unit 4, and the pulse is applied to the solenoid coil 30.

When the pulse is applied to the solenoid coil 30, a time at which the solenoid coil 30 generates a magnetic force according to the output time of the pulse, is adjusted, and a collision time at which the moving magnetic body 90 collides with the piston 40, is adjusted.

As the output time tₒₙ of the pulse is increased, the collision time of the moving magnetic body 90 and the piston 40 is increased, and the forward movement time of the piston 40 is increased and thus, the ejection time of the drug is increased, and the injection amount of the drug is increased. Thus, as the output time of the pulse is increased, the injection amount of the drug is increased, and as the output time of the pulse is decreased, the injection amount of the drug is decreased. The output time of the pulse is increased/decreased so that the user can adjust a desired drug injection amount.

In addition, the size of the magnetic force generated in the solenoid coil 30 according to the amplitude V of the pulse is adjusted, and the forward movement speed of the moving magnetic body 90 is adjusted according to the size of the magnetic force. As the amplitude of the pulse is increased, the forward movement speed of the moving magnetic body 90 is increased and thus, the injection depth of the drug is increased. Thus, as the amplitude of the pulse is increased, the injection depth of the drug is increased, and the amplitude of the pulse is decreased, the injection depth of the drug is decreased. That is, the amplitude of the pulse is increased/decreased so that the user can adjust a desired drug injection depth.

Referring to FIG. 7, it can be known that, in the 4 times burst mode, the pulse is output 4 times and the pulse is output during the second output time tₒₙ₂2 and is not output during the first output off time t_{off}1 and is generated with a waveform of the first amplitude V1.

The output time of the pulse is output as the second output time tₒₙ2 that is longer than the first output time tₒₙ1 so that the output time tₒₙ of the pulse is increased, the collision time of the moving magnetic body 90 and the piston 40 is increased, and the forward movement time of the piston 40 is increased and thus, the ejection time of the drug is increased, and the injection amount of the drug is increased. Thus, the drug can be injected with a second injection amount greater than the first injection amount.

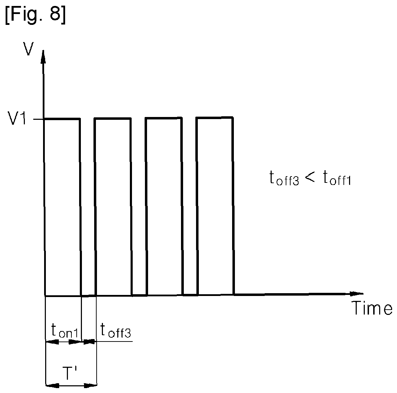

Meanwhile, FIG. 8 is a view showing a third example of a waveform of a pulse when the injection mode of the needless syringe system according to an embodiment of the present invention is a 4 times burst mode and the first injection amount and a second injection speed are set.

Referring to FIG. 8, the user sets the injection mode to a 4 times burst mode through the user interface 2, wherein the injection speed of the drug is set to a second speed that is faster than the first speed, and a case where the injection amount of the drug is set to the first injection amount and the injection depth is set to a first level, will be described.

When the 4 times burst mode is set, the control unit 4 sets a number of outputs N of the pulse output when the switch of the needleless syringe is turned on, to 4 times. That is, when the switch is turned on once, the pulse is output 4 times and thus the drug can be injected 4 times with one switching operation.

In addition, when the injection amount is set to the first injection amount through the user interface 2, the control unit 4 sets the output time tₒₙ of the pulse to a first output time tₒₙ1 according to the first injection amount. That is, the control unit 4 in which data about the relationship between the injection amount and the output time tₒₙ of the pulse are previously stored, may derive the output time tₒₙ suitable for the set injection amount when the injection amount is set.

In addition, when the injection depth is set to the first injection depth through the user interface 2, the control unit 4 sets the amplitude V of the pulse to a first amplitude V1 according to the first injection depth. That is, the control unit 4 in which data about the relationship between the injection depth and the amplitude V of the pulse are previously stored, may derive the amplitude V suitable for the set injection depth when the injection depth is set. As the injection depth is set larger, the amplitude of the pulse is set larger.

In addition, when the injection speed is set to the second injection speed that is faster than the first injection speed through the user interface 2, the control unit 4 sets a period T' of the pulse according to the second injection speed.

The control unit 4 in which data about the relationship between the injection speed and the period T of the pulse are previously stored, may derive the period T of the pulse suitable for the set injection speed when the injection speed is set. Here, the injection speed is a frequency per second, i.e., a number of injections per second, as the period of the pulse is decreased, a next pulse after the pulse is output, is output faster and thus, a number of injections per second is increased, and the injection speed is increased. Thus, the injection speed is adjusted in such a way that as the injection speed is set faster, the period of the pulse becomes shorter and as the injection speed is set slower, the period of the pulse becomes longer.

Since the injection speed is set to the second injection speed faster than the first injection speed, the period of the pulse is set to the period T' that is shorter than the period T when the injection speed is the first injection speed.

The control unit 4 sets a third output off time t_{off}3 that is the remaining time obtained by subtracting the first output time tₒₙ1 from the period T' of the pulse. The third output off time t_{off}3 is a time that is shorter than the first output off time t_{off}1.

Comparing FIG. 6 and FIG. 8, the injection amount is the same as the first injection amount, and the output time tₒₙ of the pulse is the same as the first output time tₒₙ1 but the injection speed is different from each other so that the third output off time t_{off}3 is set to be shorter than the first output off time t_{off}1.

When the output off time of the pulse is decreased, a next pulse after the pulse is output, is output faster so that a number of injections per second is increased and the injection speed is increased. Thus, the injection speed is set in such a way that as the injection speed is set faster, the period of the pulse becomes shorter and as the injection speed is set slower, the period of the pulse becomes longer. In addition, as the injection speed is set faster, the output off time t_{off} of the pulse is set shorter, and as the injection speed is set slower, the output off time t_{off} of the pulse is set longer.

As described above, when the user sets the 4 times burst mode, the injection depth of the drug, the injection amount of the drug and the injection speed of the drug, respectively, the control unit 4 sets each of the amplitude V of the pulse, the output time tₒₙ of the pulse, and the output off time t_{off} of the pulse according to the injection depth, the injection amount, and the injection speed.

The pulse generator 6 generates a pulse according to a number of outputs of the pulse, the amplitude V of the pulse, the output time tₒₙ of the pulse, and the output off time t_{off} of the pulse, which are set by the control unit 4, and the pulse is applied to the solenoid coil 30.

Referring to FIG. 7, it can be known that, in the 4 times burst mode, the pulse is output 4 times and the pulse is output during the first output time tₒₙ1 and is not output for the third output off time t_{off}3 and is generated with a waveform of the first amplitude V1.

The period T' of the pulse is shortened so that the output off time of the pulse is set to the second output off time t_{off}3 that is shorter than the first output off time t_{off}1 so that a time at which the pulse is not output, is shortened and an output interval between pulses is reduced and thus, a number of injections per second of the drug is increased, and the injection speed of the drug is faster. Thus, the drug can be injected at a faster second injection speed.

Meanwhile, the pulse for adjusting the injection amount of the drug and the injection speed in the burst mode has been described as an example with reference to FIGS. 6 through 8, but the present invention is not limited thereto and can also be applied to the continuous shot mode.

Meanwhile, in the above-described embodiments, a case where the nozzle portion opening/closing valve 50 is installed, has been described, but the present invention is not limited thereto, and there may also be no nozzle portion opening/closing valve 50. When the nozzle portion opening/closing valve 50 is not provided, the drug in the drug accommodating portion 22 is previously filled.

In addition, in the above-described embodiments, a case where a drug pressurizing portion that pressurizes the drug in the drug accommodating portion is a piston 40 and a driving unit for driving the drug pressurizing portion includes a solenoid coil 30, has been described.

However, the present invention is not limited thereto, and any drug pressurizing portion can be applied as long as it can pressurize the drug, such as an elastic membrane except for the piston 40.

In addition, the driving unit is an energy source that can apply pressure to the drug pressurizing portion such as the piston or the elastic membrane, and any driving unit that generates a driving force in a pulse form when a pulse is applied by the pulse generator, is applicable.

For example, the driving unit may include a pneumatic actuator, a hydraulic actuator, a piezo actuator, a spring device, etc.

In addition, the driving unit may also include a laser beam. That is, the driving unit may also be configured to irradiate a laser beam directly on the drug and to irradiate the laser beam on a working fluid in a separate closed pressure chamber and to generate volume expansion due to bubbles generated in the working fluid to cause the elastic membrane to be enlarged and to apply momentary pressure to the drug in the drug accommodating portion.

In addition, the driving unit may include an electrode and may be configured to generate bubbles in the working fluid in a separate closed pressure chamber due to insulation destruction together with sparks when high-voltage electricity is discharged to the electrode and to generate volume expansion due to bubbles generated in the working fluid to cause the elastic membrane to be enlarged and to apply momentary pressure to the drug in the drug accommodating portion.

While the present invention has been particularly shown and described with reference to exemplary embodiments thereof, it will be understood by those of ordinary skill in the art that various changes in form and details may be made therein without departing from the spirit and scope of the present invention as defined by the following claims.

### Industrial Applicability

According to the present invention, a needleless syringe system having adjustable drug injection attributes can be manufactured.

## Claims

1. A needleless syringe system having adjustable drug injection attributes, the needleless syringe system comprising:
a solenoid coil wound on an outer circumferential surface of the body;
a cylinder coupled to the body to be in communication with an open front surface of the body;
a drug accommodating portion, which is formed on a front inner side of the cylinder and in which a drug injected from an outside is accommodated;
a nozzle portion, which is provided in front of the cylinder and through which the drug accommodated in the drug accommodating portion is discharged forward;
a moving magnetic body, which is provided inside the body and moves forward by a magnetic force generated when power is supplied to the solenoid coil;
a piston, which is provided inside the cylinder and moves forward by an impulse applied by the moving magnetic body when the moving magnetic body moves forward, to pressurize the drug in the drug accommodating portion;
a pulse generator applying a pulse to the solenoid coil;
a user interface that allows a user to set injection attributes including at least one of an injection mode, an injection amount, an injection depth, and an injection speed of the drug discharged through the nozzle portion and injected into the skin; and
a control unit adjusting at least one of a number of outputs N, a period T, an amplitude, an output time tₒₙ, and an output off time t_{off} at which no pulse is output, of a pulse applied to the solenoid coil from the pulse generator according to the injection attributes set through the user interface to control the injection attributes.

2. The needleless syringe system of claim 1, wherein the user interface in which an injection mode of the drug is divided into a single shot mode in which the drug is injected the drug once, a burst mode in which the drug is injected at a set number of times and a continuous shot mode in which injection and blocking of the drug is continuously repeated and is displayed, allows the user to select and set the injection mode of the drug.

3. The needleless syringe system of claim 2, wherein, when the single shot mode is set through the user interface, the control unit sets a number of outputs of the pulse once, adjusts an amplitude of the pulse according to an injection depth of the drug set through the user interface and adjusts an output time tₒₙ of the pulse according to an injection amount of the drug.

4. The needleless syringe system of claim 2, wherein, when the burst mode is set through the user interface, the control unit sets a number of outputs of the pulse to the set number of times, adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}.

5. The needleless syringe system of claim 2, wherein, when the continuous shot mode is set through the user interface, the control unit adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}.

6. The needleless syringe system of claim 1, wherein the user interface allows the user to increase/decrease and to set the injection amount of the drug within a set range, and the control unit increases the output tine tₒₙ of the pulse as the injection amount of the drug set through the user interface is increased.

7. The needleless syringe system of claim 1, wherein the user interface in which the injection depth of the drug is divided into a plurality of levels at a set depth interval, allows the user to select one of the plurality of levels and set the selected level, and the control unit increases the amplitude of the pulse as the injection depth of the drug set through the user interface is increased.

8. The needleless syringe system of claim 1, wherein the user interface In which the injection speed of the drug is displayed in a number of injections per second of the drug, allows the user to set the injection speed by increasing/decreasing the number of injections per second of the drug, and as the injection speed of the drug set through the user interface is increased, the control unit decreases the period of the pulse and sets a time obtained by subtracting the output time tₒₙ of the pulse from the period of the pulse, to the output off time t_{off}.

9. The needleless syringe system of claim 1, further comprising an elastic member for a piston, which is provided inside of at least one of the body and the cylinder and applies an elastic force to the piston in a direction in which the piston moves backward, when a supply of current to the solenoid coil is cut off.

10. The needleless syringe system of claim 1, further comprising a nozzle portion opening/closing valve that is provided to open/close a passage hole between the nozzle portion and the drug accommodating portion, is pushed by a fluidic pressure applied by the drug from the drug accommodating portion during forward movement of the piston to open the passage hole, and is elastically restored when the fluidic pressure is released to close the passage hole.

11. The needleless syringe system of claim 1, further comprising a cooling chamber that is provided to surround an outside of the solenoid coil on an outside of the body and absorbs and cools heat generated in the solenoid coil through a cooling fluid.

12. A needleless syringe system having adjustable drug injection attributes, the needleless syringe system comprising:
a solenoid coil wound on an outer circumferential surface of the body;
a cylinder coupled to the body to be in communication with an open front surface of the body;
a drug accommodating portion, which is formed on a front inner side of the cylinder and in which a drug injected from an outside is accommodated;
a nozzle portion, which is provided in front of the cylinder and through which the drug accommodated in the drug accommodating portion is discharged forward;
a moving magnetic body, which is provided inside the body and moves forward by a magnetic force generated when power is supplied to the solenoid coil;
a piston, which is provided inside the cylinder and moves forward by an impulse applied by the moving magnetic body when the moving magnetic body moves forward, to pressurize the drug in the drug accommodating portion;
a nozzle portion opening/closing valve that is provided to open/close a passage hole between the nozzle portion and the drug accommodating portion, is pushed by a fluidic pressure applied by the drug from the drug accommodating portion during forward movement of the piston to open the passage hole and is elastically restored when the fluidic pressure is released to close the passage hole;
a cooling chamber that is provided to surround an outside of the solenoid coil on an outside of the body and absorbs and cools heat generated in the solenoid coil through a cooling fluid;
a pulse generator applying a pulse to the solenoid coil;
a user interface that allows a user to set injection attributes including at least one of an injection mode, an injection amount, an injection depth, and an injection speed of the drug discharged through the nozzle portion and injected into the skin; and
a control unit adjusting at least one of a number of outputs N, a period T, an amplitude, an output time tₒₙ, and an output off time t_{off} at which no pulse is output, of a pulse applied to the solenoid coil from the pulse generator according to the injection attributes set through the user interface to control the injection attributes,
wherein the user interface in which an injection mode of the drug is divided into a single shot mode in which the drug is injected the drug once, a burst mode in which the drug is injected at a set number of times and a continuous shot mode in which injection and blocking of the drug is continuously repeated and is displayed, allows the user to select and set the injection mode of the drug, and
when the single shot mode is set through the user interface, the control unit sets a number of outputs of the pulse once, adjusts an amplitude of the pulse according to an injection depth of the drug set through the user interface and adjusts an output time tₒₙ of the pulse according to an injection amount of the drug, and
when the burst mode is set through the user interface, the control unit sets a number of outputs of the pulse to the set number of times, adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}, and
when the continuous shot mode is set through the user interface, the control unit adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}.

13. A needleless syringe system having adjustable drug injection attributes, the needleless syringe system comprising:
a solenoid coil wound on an outer circumferential surface of the body;
a cylinder coupled to the body to be in communication with an open front surface of the body;
a drug accommodating portion, which is formed on a front inner side of the cylinder and in which a drug injected from an outside is accommodated;
a nozzle portion, which is provided in front of the cylinder and through which the drug accommodated in the drug accommodating portion is discharged forward;
a piston, which is provided inside the body and moves forward by a magnetic force generated when power is supplied to the solenoid coil, to pressurize the drug in the drug accommodating portion;
a pulse generator applying a pulse to the solenoid coil;
a user interface that allows a user to set injection attributes including at least one of an injection mode, an injection amount, an injection depth, and an injection speed of the drug discharged through the nozzle portion and injected into the skin; and
a control unit adjusting at least one of a number of outputs N, a period T, an amplitude, an output time tₒₙ, and an output off time t_{off} at which no pulse is output, of a pulse applied to the solenoid coil from the pulse generator according to the injection attributes set through the user interface to control the injection attributes.

14. A needleless syringe system having adjustable drug injection attributes, the needleless syringe system comprising:
a drug accommodating portion in which a drug injected from an outside is accommodated;
a nozzle portion through which the drug accommodated in the drug accommodating portion is discharged forward;
a solenoid mechanism comprising a piston that pressurizes the drug in the drug accommodating portion repeatedly while repeating forward movement and backward movement so that the drug accommodated in the drug accommodating portion is discharged through the nozzle portion, a solenoid coil that forms an electromagnetic force to move the piston forward, and a pulse generator that applies a pulse to the solenoid coil; and
a control unit controlling the pulse generator to adjust an injection amount and an injection depth of the drug discharged through the nozzle portion and injected into the skin.

15. A needleless syringe system having adjustable drug injection attributes, the needleless syringe system comprising:
a drug accommodating portion in which a drug injected from an outside is accommodated;
a nozzle portion through which the drug accommodated in the drug accommodating portion is discharged forward;
a solenoid mechanism comprising a piston that pressurizes the drug in the drug accommodating portion repeatedly while repeating forward movement and backward movement so that the drug accommodated in the drug accommodating portion is discharged through the nozzle portion, a solenoid coil that forms an electromagnetic force to move the piston forward, and a pulse generator that applies a pulse to the solenoid coil;
a user interface that allows a user to set an injection amount and an injection depth of the drug discharged through the nozzle portion and injected into the skin; and
a control unit controlling the pulse generator based on setting information input to the user interface.

16. A needleless syringe system having adjustable drug injection attributes, the needleless syringe system comprising:
a cylinder having a drug accommodating portion in which a drug injected from an outside is accommodated, formed therein;
a nozzle portion, which communicates with the drug accommodating portion of the cylinder and through which the drug accommodated in the drug accommodating portion is discharged forward;
a drug pressurizing portion pressurizing the drug in the drug accommodating portion to flow the drug toward the nozzle portion;
a driving unit driving the drug accommodating portion;
a pulse generator that applies a pulse to the driving unit;
a user interface that allows a user to set injection attributes including at least one of an injection mode, an injection amount, an injection depth, and an injection speed of the drug discharged through the nozzle portion and injected into the skin; and
a control unit adjusting at least one of a number of outputs N, a period T, an amplitude, an output time tₒₙ, and an output off time t_{off} at which no pulse is output, of a pulse applied to the driving unit from the pulse generator according to the injection attributes set through the user interface, to control the injection attributes.

17. The needleless syringe system of claim 16, wherein the user interface in which an injection mode of the drug is divided into a single shot mode in which the drug is injected the drug once, a burst mode in which the drug is injected at a set number of times and a continuous shot mode in which injection and blocking of the drug is continuously repeated and is displayed, allows the user to select and set the injection mode of the drug.

18. The needleless syringe system of claim 17, wherein, when the single shot mode is set through the user interface, the control unit sets a number of outputs of the pulse once, adjusts an amplitude of the pulse according to an injection depth of the drug set through the user interface and adjusts an output time tₒₙ of the pulse according to an injection amount of the drug.

19. The needleless syringe system of claim 17, wherein, when the burst mode is set through the user interface, the control unit sets a number of outputs of the pulse to the set number of times, adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}.

20. The needleless syringe system of claim 17, wherein, when the continuous shot mode is set through the user interface, the control unit adjusts an amplitude of the pulse according to an injection depth of the drug set through the user interface, adjusts an output time tₒₙ of the pulse according to an injection amount of the drug, adjusts a period of the pulse according to an injection speed of the drug, and sets a time obtained by subtracting the output time tₒₙ of the pulse from the period of the pulse, to the output off time t_{off}.
